# FASCICULE DE BREVET EUROPEEN

(11) **EP 2 693 995 B1**
(45) Date de publication et mention de la délivrance du brevet: **18.05.2016**
(21) Numéro de dépôt: 12714768.4
(22) Date de dépôt: 13.03.2012
(51) Int. Cl.: A61F 5/56

(54) **APPAREIL D'ORTHOPÉDIE FONCTIONNELLE DENTO-FACIALE**
FUNKTIONELLE DENTOFAZIALE ORTHOPÄDISCHE VORRICHTUNG
FUNCTIONAL DENTOFACIAL ORTHOPEDIC APPARATUS

(30) Priorité: 01.04.2011 FR 1100983
(43) Date de publication de la demande: 12.02.2014
(73) Titulaire: SO.DIS.AP.F, 34000 Montpellier (FR)
(72) Inventeur: MATHIEU, Rodrigue, 34000 Montpellier (FR)
(74) Mandataire: Schmidt, Martin Peter
(86) Numéro de dépôt international: PCT/FR2012/050523
(87) Numéro de publication internationale: WO 2012/131208

(56) Documents cités:
- EP-A1- 0 293 682
- EP-A1- 0 315 777
- FR-A1- 2 786 089
- FR-A1- 2 863 863
- FR-A1- 2 867 058

## Description

### Domaine de l'invention

La présente invention concerne un appareil d'orthopédie fonctionnelle dento-faciale visant à corriger des troubles de fonctionnement des fonctions neurovégétatives, notamment des troubles de la ventilation nasale, de la mastication, de la déglutition, de la phonation afin d'optimiser l'occlusion et de permettre le développement des bases osseuses et le repositionnement des dents dans un but fonctionnel et esthétique.

### Etat de la technique

Pour corriger la position des dents chez les patients, l'orthopédie fonctionnelle utilise généralement des appareils correcteurs amovibles. Ces correcteurs sont du type « positionneurs orthodontiques » ou du type « activateurs-gouttières», ils sont généralement réalisés dans un matériau souple et, lorsqu'ils sont introduits dans la bouche, ils exercent des pressions sur les dents mal positionnées pour corriger les bases osseuses des dents ainsi que la relation entre les deux mâchoires.

Un premier type d'appareils d'orthopédie fonctionnelle est constitué par des « positionneurs orthodontiques ». Ces appareils sont exécutés individuellement pour chaque patient. Un tel appareil est décrit dans le document EP 0 315 777 ou encore dans le document EP 0 293 682, il a une forme générale en U et comporte, de part et d'autre d'une paroi horizontale centrale, des creux de réception des dents des arcades supérieure et inférieure. L'appareil décrit dans ce document comporte également des moyens pour guider la langue comprenant une saillie pointue formée sur le côté lingual de la partie médiane inférieure de l'appareil et une zone évidée située au-dessus de la saillie pour recevoir l'extrémité de la langue. L'appareil présente comme inconvénient principal le fait de devoir placer les dents dans des emplacements précis ayant la forme des dents, les mouvements des dents à l'intérieur du positionneur n'étant pas permis. Par ailleurs, les protubérances de la partie médiane de l'appareil sont destinées à décourager une position incorrecte de la langue, mais elles ne font qu'orienter le bout de la langue par rapport à une ligne médiane de l'appareil afin de l'empêcher de prendre une position trop avancée ou trop abaissée.

Les activateurs-gouttières sont des appareils de forme et de taille standard. Le document EP 0 925 039 au nom de la demanderesse décrit un tel type d'appareil qui comprend deux gouttières qui ont une forme en U évasé, qui sont séparées par une cloison horizontale et dont la surface interne est lisse. Les parois à surface interne lisse des gouttières sont définies entre un bandeau vestibulaire d'une part et un bandeau lingual et palatin d'autre part et la cloison horizontale. Plus particulièrement selon ce document, la partie de la gouttière destinée à recevoir les incisives inférieures a la forme d'un canal droit qui débouche à ses extrémités dans deux canaux dentaires destinés à recevoir une canine inférieure, les prémolaires et au moins une molaire inférieure.

Par ailleurs, le document FR 2 641 964 décrit un autre exemple d'activateur-gouttière comportant des gouttières ayant chacune une forme semi-elliptique, leurs surfaces internes ayant des parois lisses, chaque gouttière présentant un canal de réception des incisives de forme arrondie.

Un activateur-gouttière est réalisé généralement en un matériau souple alimentaire, à base de caoutchouc naturel ou synthétique. Lorsqu'il est placé dans la cavité buccale d'un patient, l'activateur-gouttière impose une position idéale des dents tout en stimulant les muscles faciaux et buccaux au cours des divers mouvements des fonctions naturelles dento-maxillo-faciales. Les muscles sont ainsi renforcés selon la correction appliquée par l'activateur. Tout en permettant de corriger la position des dents, d'améliorer l'occlusion et le fonctionnement des muscles faciaux et buccaux, on s'est toutefois aperçu que ces appareils trouvaient leurs limites lorsque des infra-clusions ou espaces créés dans le sens vertical, entre les incisives supérieures et inférieures apparaissent lors du traitement ou lorsqu'elles existaient déjà avant le traitement. Ceci est dû principalement à une mauvaise position de la langue, associée à une déglutition atypique.

Pour essayer de remédier à ce problème, le document FR 2 867 058 a proposé un appareil de positionnement lingual doté d'une rampe linguale concave permettant à la langue d'y pendre appui et de la faire remonter vers le palais, ainsi qu'une languette positionnée à l'aplomb de la rampe linguale formant un deuxième point d'appui pour la langue. La rampe linguale est maintenue en sa partie centrale par un support de forme bombée. Un tel appareil permet, certes, de mieux orienter et stimuler la langue que les appareils précédents, mais uniquement dans la partie médiane de l'appareil.

Le but de la présente invention est de remédier au moins en partie à ces inconvénients et de proposer un appareil d'orthopédie dento-faciale qui permet une bonne orientation de la langue, et ceci dès l'introduction de l'appareil dans la cavité buccale d'un patient, tout en assurant une meilleure tonicité des tissus bucco-dentaires du patient qui utilise l'appareil.

Un autre but de l'invention est de proposer un appareil d'orthopédie dento-faciale qui assure une meilleure stimulation des muscles faciaux et buccaux pour une efficacité améliorée du traitement, tout en minimisant les risques de récidive ou d'apparition de nouvelles malpositions dentaires.

Un autre but de l'invention est de proposer un appareil d'orthopédie dento-faciale qui soit confortable à utiliser, tout en assurant un traitement efficace et fiable.

Un autre but de l'invention est de proposer un appareil d'orthopédie dento-faciale de taille standard qui puisse être facilement adapté au patient à traiter, tout en pouvant être fabriqué de manière économique en série.

### Objet de l'invention

Les buts de l'invention sont atteints avec un appareil d'orthopédie fonctionnelle dento-faciale réalisé en un matériau souple comportant deux gouttières, ayant chacune, extérieurement, en plan horizontal, une forme générale en U évasé, les deux gouttières étant séparées par une cloison sensiblement horizontale, chaque gouttière définit un canal dentaire englobant l'arcade l'alvéolo-dentaire supérieure, respectivement inférieure, ledit canal étant formé par le bandeau vestibulaire, d'une part, par le bandeau lingual, d'autre part, ainsi que par la cloison horizontale, la surface intérieure de chaque canal étant une surface lisse, du fait qu'il comprend :
- une rampe linguale continue inclinée de support et de guidage de la langue en direction du palais formée sur la partie supérieure,
- d'un relief interne renforcé ayant une forme générale en U évasé s'étendant sur le pourtour interne des gouttières et dont la section transversale augmente progressivement à partir d'une valeur minimale à l'extrémité dudit relief jusqu'à une valeur maximale dans la partie centrale de celui-ci.

L'appareil est réalisé en un matériau souple alimentaire ou biocompatible, afin que, lors du port en bouche et des exercices de mastication effectués, cet appareil puisse avoir un comportement élastique, sans blesser les muqueuses. L'appareil a une forme de double gouttière englobant en même temps les deux arcades. De par son comportement élastique, l'appareil de l'invention procure des stimulations des muscles faciaux et buccaux au cours des exercices de mastication. L'appareil retrouve toujours sa forme primitive qui est celle d'arcades idéales placées en rapport normal ou corrigé.

Les appareils de l'invention sont dédiés aux enfants et aux adultes, ils ont des formes et tailles standardisées selon l'âge du patient ou l'anomalie à traiter en rééduquant les fonctions neurovégétatives.

Selon l'invention, l'appareil d'orthopédie dento-faciale comprend une rampe linguale continue inclinée pour orienter la langue en direction du palais, la rampe étant formée sur la partie supérieure d'un relief interne renforcé qui a une forme générale en U évasé et qui s'étend sur toute ou la majeure partie du pourtour interne des gouttières. Une telle rampe forme une surface d'appui continue pour la langue, sur son pourtour. Le relief interne renforcé a donc sensiblement une même forme que les gouttières, il est réalisé sous forme d'un bourrelet continu dont le contour, tel que vu en plan horizontal, est délimité, du côté du canal dentaire, par le bandeau lingual, et à l'intérieur par un bandeau interne permettant le passage de la langue. Ce relief interne renforcé présente avantageusement une section transversale qui augmente à partir d'une valeur minimale à l'une des extrémités du relief interne (ou à l'extrémité d'une première branche latérale du U) jusqu'à une valeur maximale atteinte dans la partie centrale de celui-ci (ou au niveau de la base du U, voire au centre de cette base). L'appareil de l'invention est symétrique, ce qui fait que la valeur de la section transversale du relief interne renforcé qui atteint sa valeur maximale dans la partie centrale de celui-ci, diminue ensuite en partant de la partie centrale jusqu'à l'extrémité de la branche latérale opposée. Ainsi, l'appareil présente une robustesse accrue dans sa partie centrale de par la présence d'un bandeau interne d'épaisseur sensiblement constante formant un relief interne renforcé. Par partie centrale on comprend la zone se trouvant au milieu du canal dentaire de réception des incisives, ou la zone située sensiblement au voisinage de l'axe médian de l'appareil ou, dans certaines variantes de l'invention, dans la zone du canal dentaire qui commence au niveau des canines et s'étend entre celles-ci. Un tel relief interne permet, de par ses dimensions importantes, de former un support pour la langue, la surface d'appui permettant un guidage progressif de la langue, au niveau de ses côtés latéraux, ainsi qu'un appui de plus grande surface pour la partie centrale de la langue, tout en offrant, de par sa robustesse renforcée, une meilleure tonicité lors des stimulations de la langue, mais également des muscles faciaux et buccaux lors des exercices de mastication ou d'entraînement.

En effet, de par les dimensions importantes du relief interne et de la présence de la rampe inclinée sur le pourtour du relief interne renforcé, la langue est contrainte à prendre appui sur la rampe inclinée qui l'oriente ainsi en direction du palais, et ceci dès l'introduction de l'appareil dans la bouche. Ceci assure une meilleure ventilation nasale ainsi qu'une bonne déglutition. Par ailleurs, lorsqu'elle prend appui sur la rampe du relief interne, la langue effectue des stimulations au contact du relief interne de support en exécutant une poussée en direction des arcades dentaires, sur le pourtour du relief interne renforcé. Ces stimulations s'ajoutent à celles des muscles bucco-faciaux effectuées lors des exercices de mastications effectuées avec l'appareil, ce qui a pour résultat de stimuler et développer le tonus musculaire. Tous les muscles sollicités (abaisseurs, élévateurs et péribuccaux) s'adaptent ainsi progressivement à un fonctionnement physiologique optimisé, permettant de maintenir l'équilibre occluso-articulaire obtenu par l'utilisation de l'appareil.

Ainsi, l'appareil d'orthopédie fonctionnelle dento-faciale de l'invention permet d'aligner les dents, de les maintenir en position corrigée, tout en assurant un bon fonctionnement des fonctions neurovégétatives, notamment en rétablissant une bonne mastication, une bonne respiration par le nez, une bonne déglutition avec un bon positionnement de la langue, de la phonation, pour des bons résultats fonctionnels et esthétiques.

Avantageusement, la profondeur de chaque canal dentaire est au moins égale à la hauteur des dents qu'il englobe, jusqu'à leur collet.

La profondeur du canal dentaire est ainsi suffisante pour permettre à l'appareil d'englober les dents de l'arcade supérieure et de l'arcade inférieure depuis leur bord tranchant au moins jusqu'à leur collet lors d'un port en bouche les dents serrées. Ceci permet une bonne transmission des stimulations et excitations provenant du contact avec les dents pour une meilleure efficacité de l'appareil.

De préférence, ladite rampe linguale inclinée comprend une pente centrale sensiblement plane raccordée à deux pentes latérales adjacentes sensiblement planes.

Une telle rampe inclinée à trois pentes permet de bien orienter la langue en direction du palais, et ceci dès l'introduction en bouche de l'appareil, en guidant la langue non seulement en sa partie centrale, mais également le long de ses parties latérales. Une telle rampe comportant des surfaces sensiblement planes confère une plus grande surface de contact de la langue pour un bon confort de celle-ci lorsqu'elle est en appui sur la rampe. L'inclinaison des pentes latérales est sensiblement égale, voire légèrement supérieure, à celle de la pente centrale.

Avantageusement, ledit relief interne renforcé présente une épaisseur plus importante dans la zone du canal dentaire de l'arcade supérieure et de l'arcade inférieure destinée à recevoir les incisives et les canines.

Un tel appareil présente une zone de rigidité transversale renforcée pour conférer une action transversale plus importante et permettant aux dents de se mettre en place dans la zone du canal dentaire de l'arcade supérieure et de l'arcade inférieure destinée à recevoir les incisives et les canines, tout en incitant la langue à se mettre en place rapidement, au contact de surfaces d'appui plus larges.

De préférence, l'appareil est réalisé à base de silicone alimentaire. Ce matériau ne doit pas comporter de résidus toxiques de catalyseur ; un silicone comportant des traces de platine convient à ce titre. Un tel matériau est généralement transparent, sa composition peut toutefois inclure une base colorante à base de colorants biocompatibles (typiquement de 1 à 4% massiques). Des arômes biocompatibles peuvent également être ajoutées dans la composition du matériau afin de rendre plus agréable l'utilisation en bouche de l'appareil.

Il a été constaté lors des tests effectués en laboratoire qu'un matériau à base de silicone alimentaire qui convenait particulièrement bien pour la réalisation de l'appareil selon l'invention, devait présenter des bonnes propriétés de souplesse et de confort lors du port en bouche, tout en ayant une grande élasticité. Par ailleurs, un tel matériau doit présenter une très forte endurance et une bonne résistance à la friction, ce qui confère une bonne efficacité du traitement et, en même temps, une plus longue durée de vie à l'appareil.

Avantageusement, ledit silicone a une dureté comprise entre 50 et 60 Shore (selon ASTM D2240), et de préférence entre 53 et 57 Shore. On préfère réaliser l'appareil de l'invention en un matériau à base de silicone ayant une déformation rémanente à la compression comprise entre 1,9% et 2,9%, et de préférence entre 2,2 et 2,6%, mesurée 30 minutes après la libération de l'échantillon d'une compression de 25% pendant 70 heures. Sa résistance à la rupture est de préférence comprise entre 8 et 12 MPa (selon ASTM D882), Son allongement à la rupture (selon ASTM D412) de préférence est compris entre 820 et 1030 %, et encore plus préférentiellement entre 875 et 975%. Sa résistance au déchirement est de préférence comprise entre 46 et 60 KN/m (selon ASTM D624B), et encore plus préférentiellement entre 50 et 56 KN/m. Un matériau optimal pour la réalisation des appareils selon l'invention présente l'ensemble de ces caractéristiques mécaniques.

Dans une variante de réalisation de l'invention, la partie du canal destinée à recevoir les incisives inférieures ou supérieures a une forme de canal droit, ledit canal débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure.

Un tel appareil comportant un canal dentaire droit ou rectiligne de réception des incisives permet de réaliser une meilleure expansion transversale des arcades dentaires supérieures et inférieures, une correction des anomalies de forme des bases osseuses buccales et des malpositions dentaires correspondantes plus efficace, tout en évitant la vestibulation des incisives supérieures et inférieures.

Dans une autre variante de réalisation de l'invention, la partie du canal destinée à recevoir les incisives inférieures ou supérieures a une forme de canal arrondi, ledit canal débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure.

Un tel appareil comportant un canal dentaire arrondi de réception des incisives permet de réaliser la correction nécessaire des anomalies de forme des bases osseuses buccales et des malpositions dentaires correspondantes, tout en exerçant moins de force, selon une direction transversale, sur les dents et étant de ce fait plus confortable lors de son utilisation en bouche.

L'objet de l'invention est également atteint avec une gamme d'appareils d'orthopédie fonctionnelle dento-faciale, du fait qu'elle comprend au moins deux appareils de l'invention choisis parmi : un appareil pour expansion transversale, un conformateur, un appareil propulseur, un appareil rétropulseur, un appareil d'entraînement pour sportifs ou un appareil anti-ronflement.

Une telle gamme d'appareils de l'invention répond aux attentes en matière d'orthodontie dento-faciale car les appareils de la gamme arrivent à corriger les principales anomalies et troubles de la ventilation nasale, de la mastication, de la déglutition, de la phonation, de l'occlusion et de la forme des bases osseuses, ou peuvent être utilisés comme protection lors de l'entraînement ou des activités sportives ou encore permettent d'empêcher le ronflement et assurent une meilleure respiration lors du sommeil. Chaque appareil de la gamme peut être réalisé en plusieurs tailles selon l'âge et la morphologie du patient.

### Description des figures

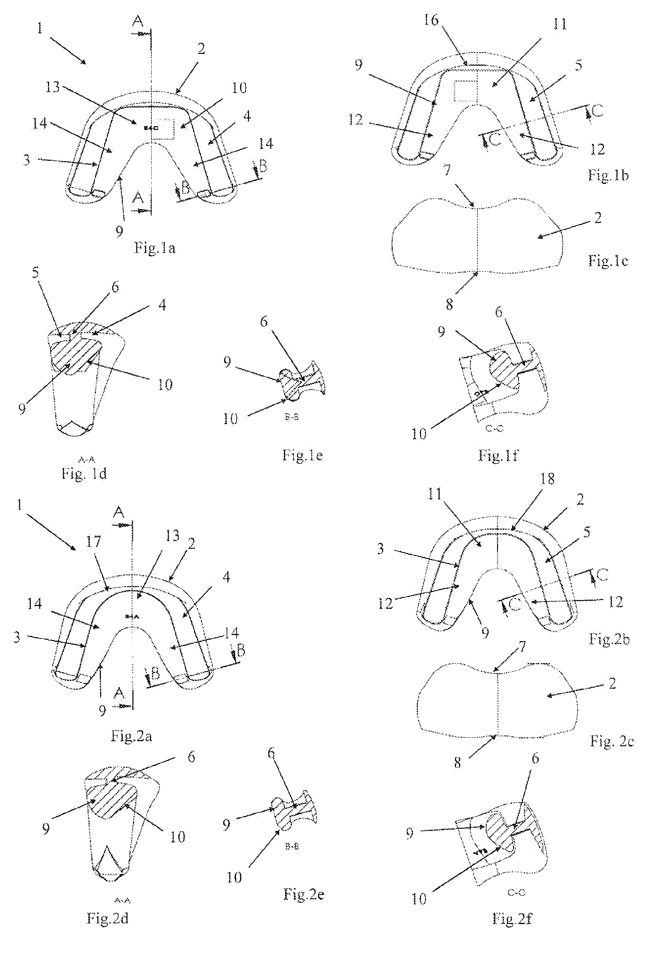

Les figures 1a à 1f illustrent un premier exemple de réalisation de l'appareil de l'invention où la figure 1 a illustre une vue de dessus, la figure 1b illustre une vue de dessous, la figure 1 c une vue frontale et les figures 1d, 1e et 1f des vues en coupe réalisées avec les plans A-A, B-B et C-C des figures 1 a et 1 b ;
Les figures 2a à 2f illustrent un deuxième exemple de réalisation de l'appareil de l'invention où la figure 2a illustre une vue de dessus, la figure 2b illustre une vue de dessous, la figure 2c une vue frontale et les figures 2d, 2e et 2f des vues en coupe réalisées avec les plans A-A, B-B et C-C des figures 2a et 2b ;

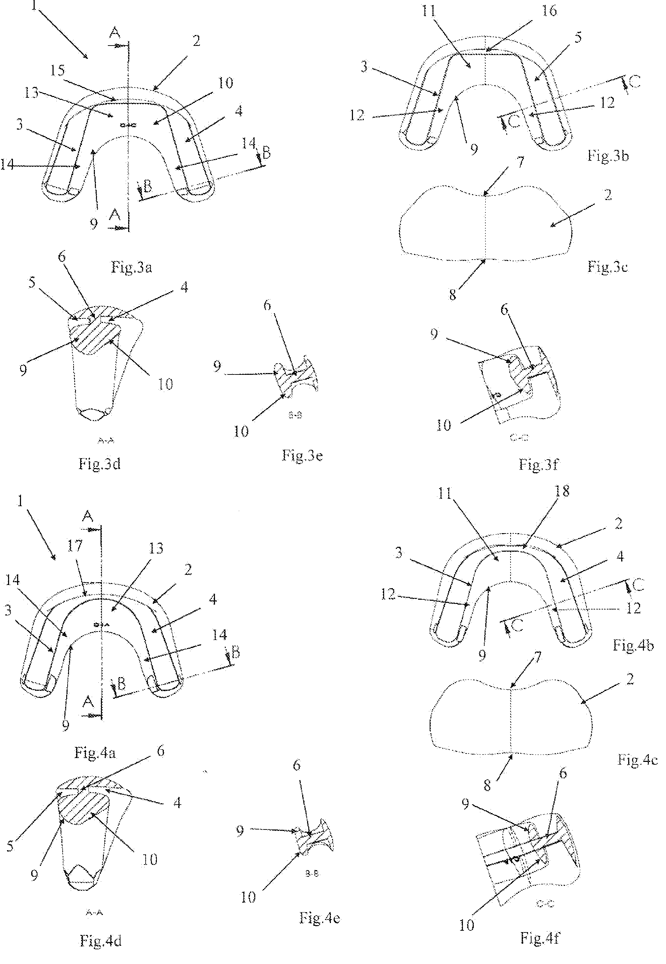

Les figures 3a à 3f illustrent un troisième exemple de réalisation de l'appareil de l'invention où la figure 3a illustre une vue de dessus, la figure 3b illustre une vue de dessous, la figure 3c une vue frontale et les figures 3d, 3e et 3f des vues en coupe réalisées avec les plans A-A, B-B et C-C des figures 3a et 3b ;
Les figures 4a à 4f illustrent un quatrième exemple de réalisation de l'appareil de l'invention où la figure 4a illustre une vue de dessus, la figure 4b illustre une vue de dessous, la figure 4c une vue frontale et les figures 4d, 4e et 4f des vues en coupe réalisées avec les plans A-A, B-B et C-C des figures 4a et 4b ;

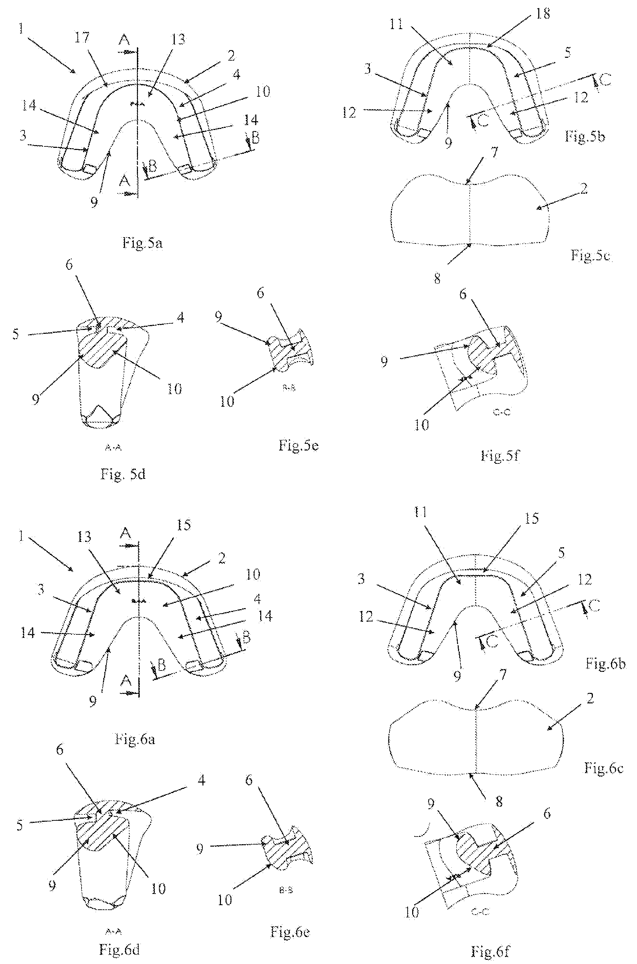

Les figures 5a à 5f illustrent un cinquième exemple de réalisation de l'appareil de l'invention où la figure 5a illustre une vue de dessus, la figure 5b illustre une vue de dessous, la figure 5c une vue frontale et les figures 5d, 5e et 5f des vues en coupe réalisées avec les plans A-A, B-B et C-C des figures 5a et 5b ;
Les figures 6a à 6f illustrent un sixième exemple de réalisation de l'appareil de l'invention où la figure 6a illustre une vue de dessus, la figure 6b illustre une vue de dessous, la figure 6c une vue frontale et les figures 6d, 6e et 6f des vues en coupe réalisées avec les plans A-A, B-B et C-C des figures 6a et 6b ;

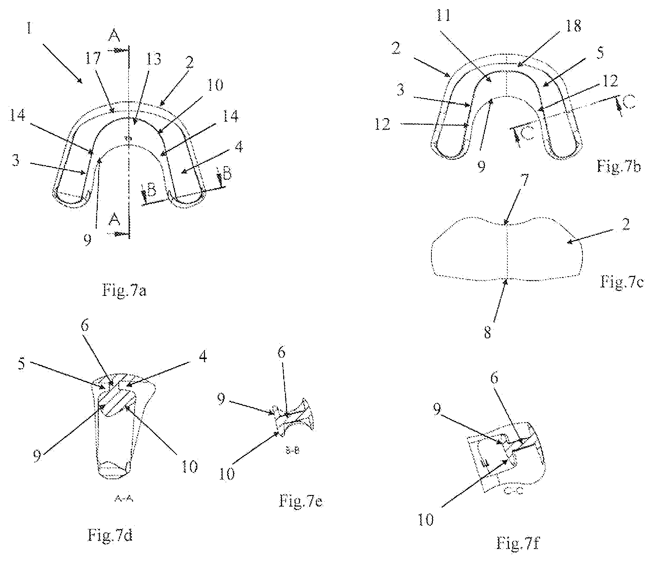

Les figures 7a à 7f illustrent un septième exemple de réalisation de l'appareil de l'invention où la figure 7a illustre une vue de dessus, la figure 7b illustre une vue de dessous, la figure 7c une vue frontale et les figures 7d, 7e et 7f des vues en coupe réalisées avec les plans A-A, B-B et C-C des figures 7a et 7b.

**Liste des repères :**

| | |
|---|---|
| 1 | Appareil d'orthodontie fonctionnelle dento-faciale |
| 2 | Bandeau vestibulaire |
| 3 | Bandeau lingual |
| 4 | Canal dentaire supérieur |
| 5 | Canal dentaire inférieur |
| 6 | Cloison horizontale |
| 7 | Echancrure supérieure |
| 8 | Echancrure inférieure |
| 9 | Relief interne renforcé |
| 10 | Rampe linguale |
| 11 | Partie centrale |
| 12 | Branche latérale |
| 13 | Pente centrale |
| 14 | Pente latérale |
| 15 | Canal droit supérieur |
| 16 | Canal droit inférieur |
| 17 | Canal arrondi supérieur |
| 18 | Canal arrondi inférieur |

### Description détaillée de l'invention

Les figures 1a à 1f illustrent un premier exemple de réalisation de l'appareil d'orthodontie fonctionnelle dento-faciale 1 de l'invention qui est un appareil correcteur pour expansion transversale, la vue de dessus 1 a correspondant à la gouttière pour la mâchoire supérieure et la vue de dessous 1 b correspondant à la gouttière pour la mâchoire inférieure. L'appareil est du type « activateur-gouttière », il a une forme générale en U, tel que vu en plan horizontal, il est symétrique par rapport à un plan médian vertical (matérialisé par le plan A-A de la figure 1 a). L'appareil comprend deux bandeaux verticaux, un bandeau vestibulaire 2 et un bandeau lingual 3 qui définissent, avec une cloison horizontale 6, un canal dentaire supérieur 4 et un canal dentaire inférieur 5. Le canal dentaire supérieur 4 et le canal dentaire inférieur 5 sont dimensionnés pour englober les arcades maxillaire et respectivement mandibulaire jusqu'au collet. La forme de la partie centrale du bandeau vestibulaire 2 est mieux visible à la figure 1c, il s'agit d'une paroi verticale de forme sensiblement rectangulaire qui atteint sa hauteur maximale en sa partie antérieure, la hauteur du bandeau vestibulaire 2 allant en diminuant au niveau des branches latérales, jusqu'à l'extrémité des gouttières. Deux échancrures sont prévues au centre du bandeau, une supérieure 7 pour le passage du frein de la lèvre supérieure et une inférieure 8 pour le passage du frein de la lèvre inférieure.

Dans le cadre des exemples ici décrits, chaque appareil peut être décliné en plusieurs tailles. Après des tests effectués en laboratoire, on a retenu un jeu de cinq ou six tailles, suivant le modèle, qui s'est avéré apte à couvrir le besoin en appareils pour enfants et adultes. Ainsi, par taille, on comprend dans ce qui suit, la réalisation d'un appareil selon l'une des cinq ou six tailles prévues par type d'exemple de réalisation. Lorsque des ajustages sont nécessaires, l'appareil d'une taille donnée peut mieux être adapté à la morphologie du patient et/ou à l'anomalie à traiter en effectuant des entailles ou en collant des surépaisseurs de matériau à certains endroits.

Plus particulièrement selon l'invention, le bandeau lingual 3 est formé à l'extrémité d'un relief interne renforcé 9 ayant une forme générale en U évasé, tel que vu en plan horizontal, dont la surface supérieure forme une rampe inclinée 10 de support et guidage de la langue en direction du palais, la section transversale dudit relief augmentant progressivement de l'extrémité vers le centre des gouttières de l'appareil. Le relief interne renforcé 9 présente une partie centrale 11 et deux branches latérales 12. Les figures 1d, 1e et 1f sont des coupes transversales montrant la forme des gouttières et l'évolution de la forme et des dimensions du relief interne renforcé 9, à partir de l'extrémité de sa branche latérale 12, mieux visible en figure 1e, au milieu de l'une de ses branches 12, tel que représenté à la figure 1f ou au milieu de la partie centrale 11, tel que visible à la figure 1 d. Le relief interne renforcé 9 a, tel que vu en coupe transversale, une forme de bourrelet arrondi à sa base, sa face supérieure étant biseautée, elle descend à partir de l'extrémité supérieure de la paroi verticale constituée par le bandeau lingual 3 et forme la rampe inclinée 10. La rampe inclinée 10 suit, en sa partie supérieure, le contour du relief interne renforcé 9, la surface de la rampe inclinée 10 qui définit la surface d'assise de la langue, augmentant progressivement de l'extrémité vers le centre des gouttières de l'appareil. La rampe inclinée 10 est formée d'une pente centrale 13 reliée à deux pentes latérales 14. La pente centrale 13 est une surface plane inclinée formée à partir de l'arrête supérieure de la partie centrale 11, elle fait un angle compris entre 30° et 60° avec l'horizontale, de préférence 45°. Les pentes latérales 14 sont des surfaces planes inclinées formées chacune à partir de l'arrête supérieure de chaque branche latérale 12 et elles font un angle compris entre 30° et 60° avec l'horizontale, de préférence 45°. Le raccordement des pentes inclinées 13 et 14 est fait en utilisant des rayons de raccordement importants de manière à obtenir une rampe à surface lisse sur tout son pourtour.

Dans l'exemple présenté aux figures 1a à 1f, la partie du canal dentaire destinée à recevoir les incisives inférieures ou supérieures a une forme de canal droit supérieur 15, respectivement inférieur 16 qui débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure. La longueur du canal droit supérieur 15 dépasse légèrement (d'environ 1 à 2mm) celle du canal droit inférieur 16. A titre d'exemple, la longueur de la base de la partie centrale 11 débouchant dans le canal droit supérieur 15 est comprise entre 28 mm et 37 mm, en fonction de la taille choisie de l'appareil.

Les figures 2a à 2f illustrent un deuxième exemple de réalisation de l'appareil d'orthodontie fonctionnelle dento-faciale 1 de l'invention qui est également un appareil correcteur pour expansion transversale. A la différence de l'appareil du premier exemple de réalisation, dans l'appareil présenté aux figures 2a à 2f, la partie du canal dentaire destinée à recevoir les incisives inférieures ou supérieures a une forme de canal arrondi supérieur 17, respectivement inférieur 18, qui débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure. La longueur du canal arrondi supérieur 17 dépasse légèrement (d'environ quelques mm) celle du canal arrondi inférieur 18. A titre d'exemple, la longueur de la base de la partie centrale 11 débouchant dans le canal droit supérieur 17 est comprise entre 27 mm et 35 mm, en fonction de la taille choisie de l'appareil.

Les appareils réalisés selon le premier et deuxième exemple de réalisation sont des appareils correcteurs pour expansion transversale dont l'action principale est des corriger les dysharmonies dento-maxillaires (les anomalies où les dents ont des dimensions trop importantes par rapport à celles des maxillaires). En outre, ces appareils sont prévus pour permettre la normalisation de l'implantation des dents, une bonne occlusion et un articulé dentaire correct et fonctionnel. Pour ceci, l'épaisseur du relief interne renforcé 9 est très importante sur tout son pourtour (plus importante que celle des autres appareils correcteurs de l'invention, tel qu'il sera expliqué par la suite) afin d'obtenir une très forte action transversale. De surcroît, l'appareil de l'invention améliore nettement la ventilation nasale et la déglutition, tout en étant d'action plus tonique.

A titre d'exemple, l'épaisseur du relief interne mesurée entre la base de la rampe inclinée 10 et la paroi verticale du bandeau lingual est comprise entre 12,75 mm et 18 mm au niveau de la partie centrale (fig. 1d et 2d) et entre 3,5 mm et 5,5 mm à l'extrémité des branches (fig. 1e et 2e), cette épaisseur varie en fonction de la taille choisie de l'appareil, plusieurs tailles étant prévues pour enfants ou adultes. Dans le cadre des mêmes exemples, la hauteur du relief interne mesurée au niveau de la partie centrale est comprise entre 18,75 mm et 25 mm.

On va maintenant décrire en référence aux figures 3a à 7f, les autres exemples d'appareils de l'invention, les parties ou éléments des appareils ayant un même rôle général ou une même forme générale que les précédents ont gardé leur numéros de référence. Ces éléments ne seront plus décrits en détail dans ce qui suit.

Les figures 3a à 3f illustrent un troisième exemple de réalisation de l'appareil d'orthodontie fonctionnelle dento-faciale 1 de l'invention qui est un appareil conformateur. Un appareil conformateur est utilisé pour agir sur les malpositions dentaires et les dysmorphoses de sens vertical, pour le traitement des béances antérieures et de la proalvéolie incisive supérieure. Un appareil conformateur est surtout utilisé en tant qu'appareil de contention, en fin de traitement, ou pour faire une pause lors du traitement, par exemple lors d'un traitement effectué avec un appareil pour expansion transversale selon l'un des exemples décrit plus haut, et a pour principale fonction de stabiliser les corrections obtenues. Dans l'appareil représenté aux figures 3a à 3f la partie du canal dentaire destinée à recevoir les incisives inférieures ou supérieures a une forme de canal droit supérieur 15, respectivement inférieur 16 qui débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure. La longueur du canal droit supérieur 15 est supérieure à celle du canal droit inférieur 16. A titre d'exemple, la longueur de la base de la partie centrale 11 débouchant dans le canal droit supérieur 15 est comprise entre 29,5 mm et 37 mm, en fonction de la taille choisie de l'appareil.

Les figures 4a à 4f illustrent un quatrième exemple de réalisation de l'appareil d'orthodontie fonctionnelle dento-faciale 1 de l'invention qui est également un appareil conformateur. A la différence de l'appareil du troisième exemple de réalisation décrit ci-dessus, dans l'appareil présenté aux figures 4a à 4f, la partie du canal dentaire destinée à recevoir les incisives inférieures ou supérieures a une forme de canal arrondi supérieur 17, respectivement inférieur 18, qui débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure. A titre d'exemple, la longueur de la base de la partie centrale 11 débouchant dans le canal droit supérieur 17 est comprise entre 27 mm et 35 mm, en fonction de la taille choisie de l'appareil.

Dans le cadre de l'appareil conformateur des figures 3a à 4f, l'épaisseur du relief interne mesurée entre la base de la rampe inclinée 10 et la paroi verticale du bandeau lingual est comprise, à titre d'exemple, entre 11,25 mm et 15 mm au niveau de la partie centrale (fig. 3d et 4d) et entre 3 mm et 4 mm à l'extrémité des branches (fig. 3e et 4e), cette épaisseur varie en fonction de la taille choisie de l'appareil, plusieurs tailles étant prévues pour enfants ou adultes. Dans le cadre des mêmes exemples, la hauteur du relief interne mesurée au niveau de la partie centrale est comprise entre 18,75 mm et 25 mm.

En comparant les dimensions de l'épaisseur du relief interne renforcé 9 des appareils conformateurs illustrés aux figures 3a à 4f à celles des appareils pour expansion transversale illustrés aux figures 1 a à 2f, on constate que le relief interne 9 de ces derniers présente des dimensions plus importantes, et donc plus de rigidité transversale que le relief des appareils conformateurs. Par conséquent, la force transversale exercée par un appareil pour expansion transversale est plus importante que celle exercée par un appareil conformateur qui ne fait, lui, que stabiliser les effets obtenus avec l'appareil de traitement pour expansion transversale utilisé.

Les figures 5a à 5f illustrent un cinquième exemple de réalisation de l'appareil d'orthodontie fonctionnelle dento-faciale 1 de l'invention qui est un appareil propulseur pour la rétromorphose ou classe Il d'Angle qui est un activateur propulseur mandibulaire, appelé dans ce qui suit propulseur. Un tel propulseur est généralement utilisé pour propulser la mandibule vers l'avant par proglissement dans la partie inférieure de l'activateur en bout à bout incisif, entraînant une adaptation anatomique des arcades et un allongement de la base mandibulaire. Le propulseur de l'invention présente un relief interne renforcé permettant, de surcroît, d'améliorer la ventilation nasale et la déglutition en assurant un bon positionnement de la langue sur la rampe inclinée du relief interne, tout en assurant une action plus tonique de l'appareil. A titre d'exemple, l'épaisseur du relief interne 9 mesurée entre la base de la rampe inclinée 10 et la paroi verticale du bandeau lingual 3 est comprise entre 13,5 mm et 18 mm au niveau de la partie centrale (fig. 5d) et entre 4,5 mm et 6 mm à l'extrémité des branches (fig. 5e), cette épaisseur varie en fonction de la taille choisie de l'appareil. Dans le cadre des mêmes exemples, la hauteur du relief interne mesurée au niveau de la partie centrale est comprise entre 18 mm et 24 mm.

Les figures 6a à 6f illustrent un sixième exemple de réalisation de l'appareil d'orthodontie fonctionnelle dento-faciale 1 de l'invention qui est un appareil rétropulseur pour antemorphose ou classe III d'Angle. L'appareil place les arcades supérieure et inférieure en rapports incisifs normaux. Il présente un bandeau vestibulaire 2 d'épaisseur plus fine en sa partie supérieure et un bandeau lingual 3 plus épais en partie supérieure, alors que l'épaisseur du bandeau vestibulaire en la partie inférieure est plus importante. L'appareil exerce une pression sagittale d'avant en arrière en prenant son point d'appui sur l'arcade supérieure. L'appareil rétropulseur de l'invention présente un relief interne renforcé permettant, de surcroît, d'améliorer la ventilation nasale et la déglutition en assurant un bon positionnement de la langue sur la rampe inclinée du relief interne, tout en assurant une action plus tonique de l'appareil. A titre d'exemple, l'épaisseur du relief interne 9 mesurée entre la base de la rampe inclinée 10 et la partie inférieure de la paroi verticale du bandeau lingual 3 est comprise entre 10,5 mm et 15 mm au niveau de la partie centrale (fig. 6d) et entre 5,5 mm et 7,5 mm à l'extrémité des branches (fig. 6e), cette épaisseur varie en fonction de la taille choisie de l'appareil. Dans le cadre des mêmes exemples, la hauteur du relief interne mesurée au niveau de la partie centrale est comprise entre 19,5 mm et 26 mm.

Les figures 7a à 7f illustrent un septième exemple de réalisation de l'appareil d'orthodontie fonctionnelle dento-faciale 1 de l'invention qui est un appareil à utiliser par les sportifs lors de l'entraînement. Cet appareil peut également être utilisé contre le ronflement lorsqu'il est utilisé pendant le sommeil, car, de par un positionnement optimum des maxillaires supérieure et inférieure, ainsi que de la langue sur le relief interne renforcé 10, il assure une bonne ventilation nasale atténuant le ronflement. A titre d'exemple, l'épaisseur du relief interne 9 mesurée entre la base de la rampe inclinée 10 et la paroi verticale du bandeau lingual 3 est comprise entre 10,88 mm et 14.2 mm au niveau de la partie centrale (fig. 7d) et entre 3 mm et 3,5 mm à l'extrémité des branches (fig. 7e), cette épaisseur varie en fonction de la taille choisie de l'appareil. Dans le cadre des mêmes exemples, la hauteur du relief interne mesurée au niveau de la partie centrale est comprise entre 15,26 mm et 19,23 mm. Les dimensions et, par conséquent, le fonctionnement de l'appareil des figures 7a à 7f se rapprochent de ceux d'un appareil conformateur à canal arrondi des figures 4a à 4f. Un tel appareil exerce moins d'effort au niveau des arcades et est donc d'une utilisation plus confortable.

L'appareil de l'invention est réalisé à base de silicone. Après des nombreux tests effectués en laboratoire, il a été établi que le matériau le plus adapté à la fabrication des appareils de l'invention était le STAMINALENE^{®} de la société STERNE. Il s'agit d'un silicone élastomère ayant des propriétés améliorées de résistance à la friction et une bonne endurance, tout en étant très confortable lorsqu'il est porté en bouche. Un tel matériau présente un ensemble optimal de caractéristiques physiques pour la réalisation de l'appareil selon l'invention, à savoir : une dureté de 55 Shore A (selon ASTM D2240), une résistance à la rupture de 10 MPa (selon ASTM D882), une résistance au déchirement de 53 kN/m (selon ASTM D624B), un allongement à la rupture de 925% (selon ASTM D412), une déformation rémanente à la compression de 2,4% mesurée 30 minutes après la libération de l'échantillon d'une compression de 25% pendant 70 heures. Sa densité est de 1,14 g/cm³.

Le matériau est transparent, il peut toutefois comprendre une base colorante utilisant des pigments biocompatibles en une proportion de 3%. Des arômes peuvent également être ajoutés à la composition afin de rendre plus agréable la mastication avec l'appareil. Ce silicone comporte un catalyseur à base de platine.

Dans un mode de réalisation avantageux de l'invention, les différents appareils peuvent être distingués par leur couleur choisie en fonction de l'utilisation de l'appareil ou du type d'anomalie ou de trouble de fonction neurovégétative à traiter avec celui-ci.

D'autres exemples et modes de réalisation de l'invention peuvent être envisagés sans sortir du cadre de ses revendications.

## Revendications

1. Appareil d'orthopédie fonctionnelle dento-faciale (1) réalisé en un matériau souple comportant deux gouttières, ayant chacune, extérieurement, en plan horizontal, une forme générale en U évasé, les deux gouttières étant séparées par une cloison sensiblement horizontale (6), chaque gouttière définit un canal dentaire (4,5) englobant l'arcade alvéolo-dentaire supérieure, respectivement inférieure, ledit canal (4,5) étant formé par un bandeau vestibulaire (2), d'une part, par un bandeau lingual (3), d'autre part, ainsi que par la cloison horizontale (6), la surface intérieure de chaque canal étant une surface lisse, et
une rampe linguale (10) continue inclinée de support et de guidage de la langue en direction du palais formée sur la partie supérieure, ladite rampe linguale (10) comprenant une pente centrale (13) sensiblement plane raccordée à deux pentes latérales (14) adjacentes sensiblement planes,
d'un relief interne renforcé (9) ayant une forme générale en U évasé s'étendant sur le pourtour interne des gouttières, et
la section transversale de ladite rampe linguale (10) augmente progressivement à partir d'une valeur minimale à l'extrémité dudit relief jusqu'à une valeur maximale dans la partie centrale de celui-ci.

2. Appareil (1) selon la revendication 1, **caractérisé en ce que** la profondeur de chaque canal dentaire (4,5) est au moins égale à la hauteur des dents qu'il englobe, jusqu'à leur collet.

3. Appareil (1) selon la revendication 2, **caractérisé en ce que** ledit relief interne renforcé (9) présente une épaisseur plus importante dans la zone du canal dentaire de l'arcade supérieure et de l'arcade inférieure destinée à recevoir les incisives et les canines.

4. Appareil (1) selon l'une des revendications précédentes, **caractérisé en ce qu'**il est réalisé à base de silicone alimentaire.

5. Appareil (1) selon la revendication 4, **caractérisé en ce que** ledit silicone a une dureté comprise entre 50 et 60 Shore, et de préférence entre 53 et 57 Shore.

6. Appareil (1) selon l'une des revendications 4 ou 5, **caractérisé en ce que** ledit silicone a une déformation rémanente à la compression comprise entre 1,9% et 2,9%, et de préférence entre 2,2% et 2,6%, mesurée 30 minutes après la libération de l'échantillon d'une compression de 25% pendant 70 heures.

7. Appareil (1) selon l'une des revendications 4 à 6, **caractérisé en ce que** la résistance à la rupture dudit silicone est comprise entre 8 et 12 MPa (mesurée selon ASTM D882), et/ou l'allongement à la rupture dudit silicone (mesuré selon ASTM D412) est compris entre 820 et 1030 %, et préférentiellement entre 875 et 975%, et/ou la résistance au déchirement dudit silicone est comprise entre 46 et 60 KN/m (mesurée selon ASTM D624B), et préférentiellement entre 50 et 56 KN/m.

8. Appareil (1) selon l'une des revendications précédentes, **caractérisé en ce que** la partie du canal destinée à recevoir les incisives inférieures ou supérieures a une forme de canal droit (15,16), ledit canal débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure.

9. Appareil (1) selon l'une des revendications 1 à 7, **caractérisé en ce que** la partie du canal destinée à recevoir les incisives inférieures ou supérieures a une forme de canal arrondi (17,18), ledit canal débouche, à ses deux extrémités, respectivement dans des canaux dentaires destinés à recevoir chacun une canine inférieure, deux prémolaires inférieures ou les deux molaires de lait correspondantes d'un enfant et, éventuellement, au moins une molaire inférieure.

10. Gamme d'appareils d'orthopédie fonctionnelle dento-faciale, **caractérisée en ce qu'**elle comprend au moins deux appareils selon l'une des revendications précédentes choisis parmi : un appareil pour expansion transversale, un conformateur, un appareil propulseur, un appareil rétropulseur, un appareil d'entraînement pour sportifs ou un appareil anti-ronflement.

## Patentansprüche

1. Funktionelle kiefer- und gesichtsorthopädische Vorrichtung (1), die aus einem weichen Material hergestellt ist, umfassend zwei Schienen, die jeweils außen auf horizontaler Ebene eine allgemeine Form eines ausgeweiteten U haben, wobei die zwei Schienen durch eine im Wesentlichen horizontale Trennwand (6) getrennt sind, wobei jede Schiene einen Dentalkanal (4, 5) definiert, der den oberen bzw. den unteren Alveolarbogen einschließt, wobei der besagte Kanal (4, 5) von einerseits einem vestibulären Band (2) und andererseits von einem lingualen Band (3) sowie der horizontalen Trennband (6) gebildet wird, wobei die Innenfläche jedes Kanals eine glatte Fläche ist, und
eine kontinuierliche geneigte linguale Rampe (10) zur Stützung und Führung der Zunge in Richtung des Gaumens, die auf dem oberen Teil ausgebildet ist, wobei die besagte linguale Rampe (10) eine im Wesentlichen ebene zentrale Schräge (13) umfasst, die mit zwei im Wesentlichen ebenen, angrenzenden seitlichen Schrägen (14) verbunden ist,
ein verstärktes inneres Relief (9) mit einer allgemeinen Form eines ausgeweiteten U, das sich auf dem inneren Umfang der Schienen erstreckt, und
wobei der Querschnitt der besagten lingualen Rampe (10) allmählich von einem minimalen Wert am Ende des besagten Reliefs zu einem maximalen Wert im zentralen Teil davon zunimmt.

2. Vorrichtung (1) nach Anspruch 1, **dadurch gekennzeichnet, dass** die Tiefe jedes Dentalkanals (4, 5) mindestens gleich der Höhe von Zähnen, die er einschließt, bis zu ihrem Hals ist.

3. Vorrichtung (1) nach Anspruch 2, **dadurch gekennzeichnet, dass** das besagte verstärkte innere Relief (9) eine Dicke aufweist, die in der Zone des Dentalkanals des oberen Bogens und des unteren Bogens größer ist und dazu vorgesehen ist, die Schneidezähne und die Eckzähne aufzunehmen.

4. Vorrichtung (1) nach einem der vorhergehenden Ansprüche, **dadurch gekennzeichnet, dass** sie auf der Basis von lebensmittelgeeignetem Silikon hergestellt wird.

5. Vorrichtung (1) nach Anspruch 4, **dadurch gekennzeichnet, dass** das besagte Silikon eine Härte zwischen 50 und 60 Shore und vorzugsweise zwischen 53 und 57 Shore aufweist.

6. Vorrichtung (1) nach einem der Ansprüche 4 oder 5, **dadurch gekennzeichnet, dass** das besagte Silikon eine bleibende Druckverformung zwischen 1,9 % und 2,9 % und vorzugsweise zwischen 2,2 % und 2,6 % aufweist, die 30 Minuten nach dem Abziehen des Prüflings von einer Druckbelastung von 25 % für 70 Stunden gemessen wird.

7. Vorrichtung (1) nach einem der Ansprüche 4 bis 6, **dadurch gekennzeichnet, dass** die Reißfestigkeit des besagten Silikons zwischen 8 und 12 MPa (gemäß ASTM D882 gemessen) liegt und/oder die Reißdehnung des besagten Silikons (gemäß ASTM D412 gemessen) zwischen 820 und 1030 % und vorzugsweise zwischen 875 und 975 % liegt und/oder die Rissfestigkeit des besagten Silikons zwischen 46 und 60 KN/m (gemäß ASTM D624B) und vorzugsweise zwischen 50 und 56 KN/m liegt.

8. Vorrichtung (1) nach einem der vorhergehenden Ansprüche, **dadurch gekennzeichnet, dass** der Teil des Kanals, der dazu vorgesehen ist, die unteren oder die oberen Schneidezähne aufzunehmen, eine Form eines geraden Kanals (15, 16) hat, wobei der besagte Kanal an seinen beiden Enden jeweils in Dentalkanälen mündet, die dazu vorgesehen sind, jeweils einen unteren Eckzahn, zwei untere vordere Backenzähne oder zwei entsprechende Milchbackenzähne eines Kinds und gegebenenfalls mindestens einen unteren Backenzahn aufzunehmen.

9. Vorrichtung (1) nach einem der Ansprüche 1 bis 7, **dadurch gekennzeichnet, dass** der Teil des Kanals, der dazu vorgesehen ist, die unteren oder die oberen Schneidezähne aufzunehmen, eine Form eines gerundeten Kanals (17, 18) hat, wobei der besagte Kanal an seinen beiden Enden jeweils in Dentalkanälen mündet, die dazu vorgesehen sind, jeweils einen unteren Eckzahn, zwei untere vordere Backenzähne oder zwei entsprechende Milchbackenzähne eines Kinds und gegebenenfalls mindestens einen unteren Backenzahn aufzunehmen.

10. Serie von funktionellen kiefer- und gesichtsorthopädischen Vorrichtungen, **dadurch gekennzeichnet, dass** sie mindestens zwei Vorrichtungen nach einem der vorhergehenden Ansprüche umfasst, die aus den folgenden ausgewählt sind: einer Vorrichtung zur transversalen Expansion, einen Konformator, eine Vorschubvorrichtung, eine Rückzugsvorrichtung, eine Trainingsvorrichtung für Sportler oder eine Antischnarchvorrzchtung.

## Claims

1. Functional orthodontic appliance (1) made of a flexible material and comprising two grooves, each having, externally, in the horizontal plane, an overall flared U shape, the two grooves being separated by a substantially horizontal partition (6), each groove defines a dental canal (4, 5) encompassing the upper or lower alveolo-dental arch, respectively, said canal (4, 5) being formed by a vestibular strip (2) and a lingual strip (3), as well as the horizontal partition (6), the inner surface of each canal being a smooth surface, and
- a continuous inclined lingual ramp (10) for supporting and guiding the tongue towards the palate formed on the upper portion, said lingual ramp (10) comprising a substantially plane central slope (13) connected to two substantially plane adjacent lateral slopes (14),
a reinforced inner raised portion (9) having an overall flared U shape extending on the inner circumference of the grooves, and
the cross-section of said lingual ramp (10) increases progressively from a minimum value at the end of said raised portion to a maximum value in the central portion thereof.

2. Appliance (1) according to claim 1, **characterised in that** the depth of each dental canal (4, 5) is at least equal to the height of the teeth encompassed therein, up to the neck thereof.

3. Appliance (1) according to claim 2, characterised that said reinforced inner raised portion (9) is thicker in the area of the dental arch of the upper arch and the lower arch intended to receive the incisors and the canines.

4. Appliance (1) according to any one of the preceding claims, **characterised in that** it is made of a food-grade silicone base.

5. Appliance (1) according to claim 4, **characterised in that** said silicone has a hardness between 50 and 60 Shore, and preferably between 53 and 57 Shore.

6. Appliance (1) according to any one of claims 4 or 5, **characterised in that** said silicone has a residual compressive deformation between 1.9% and 2.9%, and preferably between 2.2% and 2.6%, measured 30 minutes after the release of the sample from 25% compression for 70 hours.

7. Appliance (1) according to any one of claims 4 to 6, **characterised in that** the rupture strength of said silicone is between 8 and 12 MPa (measured as per ASTM D882), and/or the elongation at break of said silicone (measured as per ASTM D412) is between 820 and 1030%, and preferentially between 875 and 975%, and/or the tear strength of said silicone is between 46 and 60 kN/m (measured as per ASTM D624B), and preferentially between 50 and 56 kN/m.

8. Appliance (1) according to any one of the preceding claims, **characterised in that** the portion of the canal intended to receive the lower or upper incisors has a straight canal (15, 16) shape, said canal opens, at both ends thereof, respectively in dental canals intended to each receive a lower canine, two lower premolars or a child's two corresponding primary molars and, optionally, at least one lower molar.

9. Appliance (1) according to any one of claims 1 to 7, **characterised in that** the portion of the canal intended to receive the lower or upper incisors has a rounded canal (17, 18) shape, said canal opens, at both ends thereof, respectively in dental canals intended to each receive a lower canine, two lower premolars or a child's two corresponding primary molars and, optionally, at least one lower molar.

10. Range of functional orthodontic appliances, **characterised in that** it comprises at least two appliances according to any one of the preceding claims chosen from: a transverse expansion appliance, a shaper, a propulsion appliance, a retropulsion appliance, a sports training appliance or an anti-snoring appliance.
